Europäisches Patentamt

European Patent Office

Office européen des brevets

(19)

(11) Veröffentlichungsnummer: **0 316 266**
**A1**

## (12) EUROPÄISCHE PATENTANMELDUNG

(21) Anmeldenummer: 88810539.2

(22) Anmeldetag: 09.08.88

(51) Int. Cl.⁴: **A 61 L 9/12**

(30) Priorität: 31.03.88 CH 1216/88

(43) Veröffentlichungstag der Anmeldung:
17.05.89 Patentblatt 89/20

(84) Benannte Vertragsstaaten: **ES GR**

(71) Anmelder: **Weick, Heinz Hermann**
**94, rue de la Servette**
**CH-1202 Genf (CH)**

(72) Erfinder: **Weick, Heinz Hermann**
**94, rue de la Servette**
**CH-1202 Genf (CH)**

(74) Vertreter: **Lauer, Joachim**
**Hug Interlizenz AG Austrasse 44 Postfach**
**CH-8045 Zürich (CH)**

(54) **Vorrichtung zur Abdunstung von Wirkstoffen.**

(57) Die Vorrichtung dient zum Abdunsten von Wirkstoffen paramedizinischer und kosmetischer Art. Sie besteht aus einem plättchenförmig flachen Hohlkörper (1,2) mit zwei Breitseitenwandungen (1b,1b'), von denen wenigstens eine einen porös ausgebildeten Bereich aufweist und mit einem auf diesem gleitend verschiebbar angeordneten, schieberartigen Abdeckorgan (5). Um die Vorrichtung gegen Wirkstoffverlust abzudichten ist der Hohlkörper mit einer umlaufenden, zum Abdeckorgan hin offenen, sich nach aussen erweiternden Einschubdichtungsnute (2b) versehen. Das Abdeckorgan ist zum Zwecke seines Eingriffs mit einem Randabschnitt in diese Einschubdichtungsnute in seiner Verschlussstellung an diesem Randabschnitt aussenseitig mit einer zur Einschubdichtungsnute gegengleichen Verjüngung (5b) versehen. Weiter sind die äussere Oberfläche des Hohlkörpers im Bereich der Einschubdichtungsnute und die innere Oberfläche des Abdeckorgans zumindest im Bereich seines Randabschnitts als glatte, in der Verschlussstellung dicht aneinander anliegende Dichtflächen ausgebildet.

Die Vorrichtung dient sowohl für eine aktive Abdunstung z.B. von paramedizinischen Wirkstoffen (Verwendung als Mund- und Nasen-Inhalator) als auch für eine passive Abdunstung z.B. von Parfum (als Duftstoffspender in Taschen von Kleidungsstücken oder als Sockelgerät).

Fig.1

## Beschreibung

### Vorrichtung zur Abdunstung von Wirkstoffen

#### Technisches Gebiet

Die Erfindung betrifft eine Vorrichtung zur Abdunstung von Wirkstoffen. Sie betrifft insbesondere eine solche Vorrichtung mit einem plättchenförmig flachen Hohlkörper mit zwei Breitseitenwandungen, von denen wenigstens eine einen porös ausgebildeten Bereich aufweist und mit einem auf diesem gleitend verschiebbar angeordneten, schieberartigen Abdeckorgan, welches aus einer den porösen Bereich des Hohlkörpers abdeckenden Verschlussstellung in wenigstens eine, diesen Bereich zumindest teilweise freigebende Abdunstungsstellung verstellbar ist.

Eine solche Vorrichtung dient der passiven Abdunstung (z.B. von Parfum), sowie der aktiven Abdunstung (z.B. von paramedizinischen Werkstoffen gegen Erkältungsbeschwerden, zum Entwöhnen des Rauchens oder dergl.). Für eine aktive Abdunstung wird die Vorrichtung vor den Mund oder unter die Nase gehalten, um den Wirkstoff durch den porösen Abdunstbereich zu inhalieren.

Da es sich bei den Wirkstoffen meist um leicht verflüchtigende Flüssigkeiten mit teilweise recht hohem Dampfdruck handelt, zwischen der Fabrikation der Geräte und deren Erwerb durch den Endverbraucher mitunter ein grösserer Zeitraum liegt, und die Verwendungsdauer möglichst gross sein soll, ist das Verlust- bzw. Dichtigkeitsproblem von grosser Bedeutung. Eine einwandfreie Abdichtung ist auch deshalb erforderlich, weil die Gesetzgeber vieler Länder eine Produktstabilität verlangt, die je nach Land zwischen 2 - 5 Jahren betragen kann. Unter Stabilität ist im Hinblick auf Abdunstungsvorrichtungen der hier betrachteten Art insbesondere zu verstehen, dass sich die in der Vorrichtung befindliche Flüssigkeit in der gesetzlich vorgeschriebenen Zeitspanne nicht verändert, also auch nicht entweichen kann. Wenn aus irgendeinem Grunde die vorgeschriebene Zeitspanne nicht garantiert werden kann, muss das Produkt mit einem Verfalldatum versehen werden, genau wie bei Lebensmitteln.

#### Stand der Technik

Es ist bisher eine Vorrichtung eingangs beschriebener Bauweise bekannt geworden. Bei dieser konnte das Problem der Gasdichtigkeit nicht zufriedenstellend gelöst werden. Insbesondere schliesst das Abdeckorgan in seiner Verschlussstellung nicht genügend gasdicht ab. Man hat daher den Abdichtbereich zwischen dem Hohlkörper und dem Abdeckorgan mit einem selbstklebenden Dichtungsband aus Aluminiumfolie umgeben, die verhältnismässig teuer und auf Grund der flachen Geräteform nur schwer zu plazieren ist. Die dadurch erzielte Verbesserung der Dichtigkeit erwies sich jedoch als ausgesprochen ungenügend. Eine ausreichend gasdichte Verklebung ist mit Selbstklebeband nicht erreichbar. Zum Ausgleich der Oberflächenrauhigkeit von Hohlkörper und Abdeckorgan muss viel Kleber verwendet werden, was nach dem Entfernen des Dichtungsbandes auf der Vorrichtung unerwünschte Rückstände zur Folge hat. Die durch das Dichtungsband erzielte Verbesserung der Dichtigkeit ist weiter nur bis zur erstmaligen Geräteverwendung durch den Verbraucher gegeben. Der anschliessend wieder auftretende höhere Verlust verkürzt nach wie vor erheblich die Zeitspanne der Benützung. Der hohe Preis der Wirkstoffe macht diesen Zustand noch unbefriedigender: dem Kunden steht nur ein Teil von dem von ihm bezahlten Inhalt zur Verfügung.

#### Darstellung der Erfindung

Der Erfindung liegt insbesondere die Aufgabe zugrunde, bei einer Vorrichtung der eingangs genannten Art, die Dichtigkeit mit möglichst einfachen Mitteln zu erhöhen. Diese sowie weitere Aufgaben werden gemäss der vorliegenden Erfindung gelöst durch eine Vorrichtung mit den Merkmalen des Patentanspruchs 1.

Erfindungsgemäss ist demnach der Hohlkörper mit einer umlaufenden, zum Abdeckorgan hin offenen, sich nach aussen erweiternden Einschubdichtungsnute und das Abdeckorgan zum Zwecke seines Eingriffs mit einem Randabschnitt in diese Einschubdichtungsnute in seiner Verschlussstellung an diesem Randabschnitt aussenseitig mit einer zur Einschubdichtungsnute gegengleichen Verjüngung versehen. Weiter sind zumindest die äussere Oberfläche des Hohlkörpers im Bereich der Einschubdichtungsnute und zumindest die innere Oberfläche des Abdeckorgans im Bereich seines Randabschnitts als glatte, in der Verschlussstellung dicht aneinander anliegende Dichtflächen ausgebildet.

Wird das auf dem Hohlkörper gleitende Abdeckorgan in seine Verschlussposition geschoben, so gelangt dessen verjüngter Randabschnitt in die Einschubdichtungsnute. Das sich diese nach innen zu verjüngt, kann der Randabschnitt ab einer gewissen Einschubstellung nur noch unter elastischer Verformung seiner selbst sowie hauptsächlich der Einschubdichtungsnute weiter in diese hineingeschoben werden. Dabei werden die genannten Dichtflächen am Hohlkörper sowie am Abdeckorgan elastisch aufeinandergepresst, wodurch sich eine ausgezeichnete Dichtwirkung ergibt.

Gemäss einer bevorzugten Ausgestaltung der Erfindung ist der Hohlkörper im Bereich der Einschubdichtungsnute und seiner darin angeordneten Dichtfläche ringsherum geringfügig verstärkt, so dass die genannte Dichtfläche zumindest gegenüber dem porösen Bereich des Hohlkörpers etwas erhaben ist. Beim Einschieben des Abdeckorgans mit seinem Randbereich in die Einschubdichtungsnute fährt dieser Randbereich auf die geringfügig erhöhte Dichtfläche auf. Die Erhöhung der Dichtfläche bewirkt in vorteilhafter Weise eine gewisse Erhöhung der elastischen Spannung, mit der die Dichtflächen am Hohlkörper im Bereich der Einschubdichtungsnute sowie am Abdeckorgan an dessen Randabschnitt in der Verschlussstellung aufeinander gepresst werden und garantiert darüberhinaus, dass diese Flächen überall einwandfrei aneinander anliegen. Weiter wird durch diese Erhöhung erreicht, dass das Abdeckorgan über dem übrigen, insbesondere dem porös ausgebildeten Bereich des Hohlkörpers leichtgängig verschieblich ausgebildet sein kann.

Eine weitere bevorzugte Ausgestaltung der Erfindung liegt darin, dass wenigstens die porös ausgebildete Breitseitenwandung des Hohlkörpers und die auf dieser gleitende Wandung des Abdeckorgans durch winzige Abstandsgleitrippen auf Abstand gehalten sind, welche eine geringere Höhe aufweisen, als die Dichtfläche am Hohlkörper zumindest gegenüber dessen porösen Bereich erhaben ist. Diese Abstandsgleitrippen verhindern, dass ein z.B. auf dem Wege der Kondensierung zwischen Abdeckorgan und Breitseitenwandung gelangendes Wirkstofftröpfchen infolge der Verschiebung des Abdeckorgans zu einer flächigen Benetzung führen kann.

Gemäss einer weiteren bevorzugten Ausgestaltung der Erfindung ist der Hohlkörper aus einem ersten und einem zweiten Teil zusammengesetzt, wobei der zweite Teil den ersten Teil mit einem Endabschnitt umgreift, und wobei die Einschubdichtungsnute durch eine innenseitige Verjüngung am genannten Endabschnitt des zweiten Teils des Hohlkörpers gebildet wird. Bei einer solchen zweiteiligen Ausführung des Hohlkörpers sind vorzugsweise dessen erster und zweiter Teil in ihrem Überlappungsbereich mittels einer umlaufenden Schweissnaht miteinander verschweisst. Zur Herstellung der Schweissnaht ist dabei weiter vorzugsweise der erste Teil des Hohlkörpers umfangsseitig mit einer umlaufenden dünnen Schweissrippe versehen, die in einen etagenförmigen Absatz im Endabschnitt des zweiten Teils des Hohlkörpers eingreift. Die Schweissnaht sollte um nicht mehr als die Tiefe der Einschubdichtungsnute von dieser entfernt angeordnet ist. Insbesondere durch eine solche Lage der Schweissnaht ergibt sich im Bereich der Einschubdichtungsnute eine erhebliche Versteifung, welche sich durch Erhöhung der vorgenannten elastischen Spannungen in der Verschlussstellung des Abdeckorgans weiter verstärkend auf die Dichtigkeit auswirkt.

Die vorgenannten sowie weitere vorteilhafte Ausgestaltungen und Weiterbildungen der vorliegenden Erfindung sind in den abhängigen Ansprüchen gekennzeichnet.

Kurze Beschreibung der Zeichnungen

In der Zeichnung ist ein Ausführungsbeispiel der Erfindung dargestellt, aus dessen Beschreibung die durch die Erfindung erzielten Vorteile sowie auch die vorstehend erwähnten bevorzugten Ausgestaltungen und Weiterbildungen klar hervorgehen. Es zeigen:

Fig. 1 einen teilgeschnittenen Seitenriss der Vorrichtung,
Fig. 2 einen Schnitt A-A nach Fig. 1,
Fig. 3 einen Schnitt B-B nach Fig. 1,
Fig. 4 einen Schnitt C-C nach Fig. 1 und
Fig. 5 einen Schnitt D-D nach Fig. 1.

In den einzelnen Figuren sind übereinstimmende Teile mit gleichen Bezugzeichen versehen.

Zur Erzielung einer grösstmöglichen Klarheit der einzelnen Konstruktionsdetails wurden die Figuren in verschiedenen Massstäben dargestellt. Demgegenüber ist die Vorrichtung in der Ausbildung zum Tragen in Taschen von Kleidungsstücken so bemessen, dass sie z.B. auch in einer kleinen Brusttasche eines Hemdes oder einer Bluse Platz findet. Für Sockelgeräte ist die Grösse von weniger grosser Bedeutung.

Bester Weg zur Ausführung der Erfindung

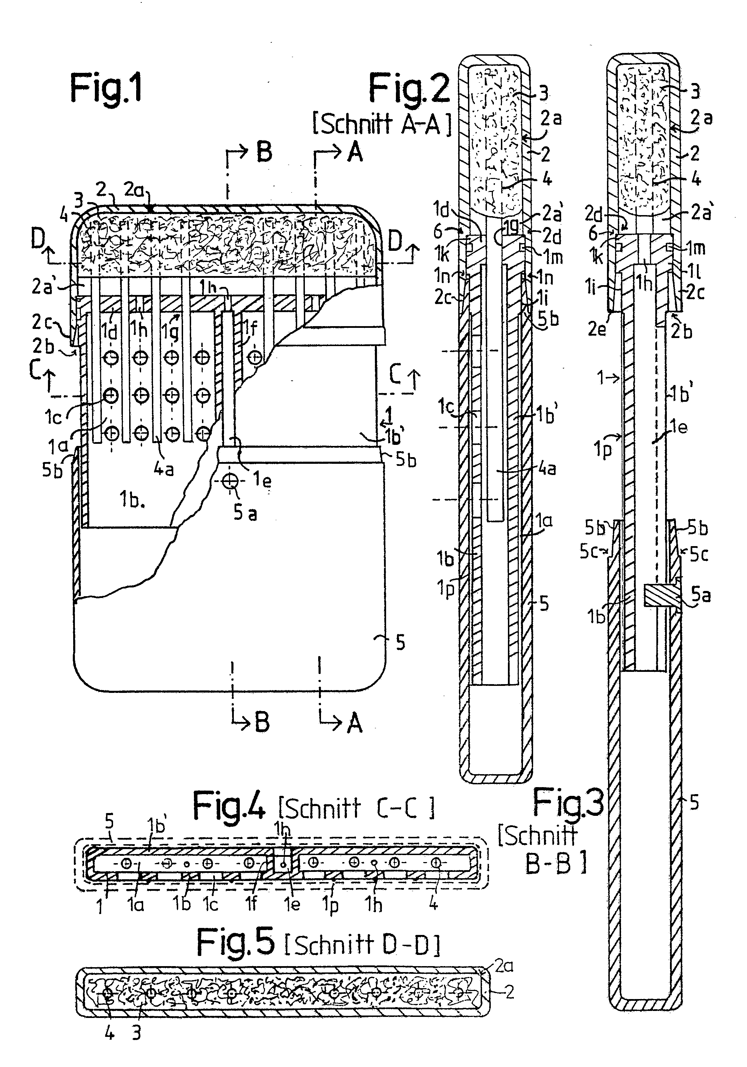

Es wird nunmehr auf die Zeichnungen Bezug genommen. Der darin dargestellte plättchenförmig flache Hohlkörper 1,2 besteht aus dem eine Abdunstkammer 1a umschliessenden ersten Teil 1 (Abdunstkammerteil) und dem zugleich als Griffteil dienenden, eine Wirkstoffreservekammer 2a enthaltenden zweiten Teil 2 (Reservekammerteil). Diese beiden Teile sind durch eine umlaufende Ultraschallverschweissung gegeneinander befestigt. Die Reservekammer 2a ist bis auf den schmalen Freiraum 2a' von einem hülsenförmig umschlossenen Tampon 3 aus saugfähigem Material ausgefüllt. Abdunstkammer 1a und Reservekammer 2a sind durch eine Querwand 1d voneinander getrennt, die an das in den zweiten Teil 2 hineinragende Ende des ersten Teiles 1, angeformt ist. In der Querwand 1d sind Durchtrittskanäle 1g vorgesehen. Durch diese führen dochtartige Wirkstoffleiter 4. Die dochtartigen Wirkstoffleiter 4 ragen mit ihren in den Figuren 1 - 3 oberen Längsbereichen in den Tampon 3. Mit ihren in den genannten Figuren unteren Bereichen 4a erstrecken sie sich in die Abdunstkammer 1a hinein und übernehmen dort unmittelbar die Abdunstfunktion. Sie sind von im wesentlichen formstabiler, gradliniger Ausbildung und bestehen vorzugsweise aus Chemiefasern.

In der Querwand 1d befinden sich noch Druckausgleichsöffnungen 1h, die zudem zum Auffüllen der Reservekammer 2a mittels Einspritzkanülen dienen. Die eine 1b der beiden Breitseitenwandungen 1b, 1b' des Abdunstkammerteiles 1 ist mit in Reihen angeordneten Abdunstlöchern 1c versehen und dadurch porös ausgebildet. Das Abdeckorgan 5 ist als schieberartiger, den Abdunstkammerteil 1 des Hohlkörpers 1,2 eng umschliessender, auf diesem gleitender Körper ausgebildet. Er besitzt zur Begrenzung seiner maximalen Auszugsposition innenseitig einen mittig angeordneten zylindrischen Anschlagnocken 5a, der in eine Anschlaglängsnute 1e des Abdunstkammerteils 1 eingreift. Diese Nute wird beidseitig durch Stege 1f begrenzt und weist keine Verbindung zur Abdunstkammer 1a auf.

Die Längsbereiche 4a der Wirkstoffleiter 4 weisen einen gewissen Abstand von den Seitenwandungen 1b, 1b' des Abdunstkammerteiles 1 auf. Dadurch ist eine unerwünschte Benetzung der genannten Wandungen verhindert. Damit die Wirkstoffleiter 4 in dieser Lage verharren, sind sie, wie schon erwähnt, im wesentlich formstabil und gradling ausgebildet und jeweils geschützt zwischen den Längsreihen der Abdunstlöcher 1c angeordnet. Zudem sichern die Stege 1f des Abdunstkammerteiles 1 den gegenseitigen Abstand der Wandungen 1b, 1b'

Nicht unbedingt erforderlich ist allerdings, dass die Wirkstoffleiter absolut keine Berührung mit den Seitenwandungen aufweisen. Bei einem bevorzugten Abstand von nur einigen 1/10 mm ist eine gewisse gegenseitige Berührung, beispielsweise schon infolge geringfügiger Durchbiegung der Wirkstoffleiter, nicht immer ganz zu vermeiden. Eine solche Berührung bleibt jedoch zumindest dann ohne nachteilige Folgen und ist tolerierbar, wenn sie mehr oder weniger nur punktuell ist.

Der Uebergangsbereich zwischen dem Abdunstkammerteil 1 und dem Reservekammerteil 2, sowie die zwischen Hohlkörper 1,2 und Abdeckorgan 5 in dessen Verschlussposition dichtend zusammenwirkenden Bereiche besitzen eine ganz spezielle, neuartige Ausbildung. So weist zunächst der Reservekammerteil 2 im Bereich seines den Abdunstkammerteil 1 umgreifenden Endabschnitts zur Bildung einer umlaufenden, zum Abdeckorgan 5 hin offenen, sich nach aussen erweiternden Einschubdiohtungsnute 2b die innenseitige, konusartige Verjüngung 2c auf. Zum Zwecke des Eingriffs in diese ist der Randabschnitt bzw. Oeffnungsbereich des Abdeckorgans 5 aussenseitig mit der gegengleichen konusartigen Verjüngung 5b versehen.

Die äussere Oberfläche des Abdunstkammerteils ist im Bereich der Einschubdichtungsnute und die innere Oberfläche des Abdeckorgans zumindest im Bereich seines Randabschnitts als glatte Dichtfläche ausgebildet. In der Verschlussstellung (Fig. 2) liegen die genannten Dichtflächen dicht aneinander an. Die Rauhtiefe der genannten Dichtflächen beträgt zwischen etwa 2/1000 mm und etwa 3/1000 mm. Des weiteren ist die Wandstärke des Abdunstkammerteils 1 im Bereich 1i der Einschub dichtungsnute 2b und damit der Dichtfläche ringsherum geringfügig, vorzugsweise um einige wenige 1/10 mm, verstärkt.

Auch die konusartig verjüngten Flächen 2c und 5b sind als entsprechend glatte Dichtflächen ausgebildet.

An den verjüngten Randbereich 5b des Abdeckorgans 5 schliesst sich ein nach aussen springender Absatz 5c an, welcher in der Verschlussstellung des Abdeckorgans, wenn dessen genannter Randbereich in die Einschubdichtungsnute 2b eingreift, am Oeffnungsrand 2e der Einschubdichtungsnute anliegt. Der Absatz 5c ist so angeordnet, dass das Abdeckorgan mit seinem Randabschnitt zum Erreichen der Verschlussstellung etwas in die Einschubdichtungsnute unter gewisser elastischer Verformung derselben hineingedrückt werden muss. Die Tiefe der Einschubdichtungsnute 2b ist derart bemessen, dass in dieser Stellung in ihrem hinteren Bereich noch ein kleiner umlaufender Hohlraum 1n verbleibt.

Die umlaufende Ultraschallverschweissung ist mit 6 bezeichnet. Sie ist um nicht mehr als die Tiefe der Einschubdichtungsnute von dieser entfernt angeordnet. Dadurch ergibt sich eine gewünscht hohe Steifigkeit des Reservekammerteils 2 im Bereich der Einschubdichtungsnute 2b. Zur Herstellung der Schweissnaht ist an den Abdunstkammerteil 1 umfangsseitig ringsherum eine umlaufende dünne Schweissrippe 1k angeformt.

Diese liegt an dem etagenförmigen Absatz 2d des Reservekammerteiles 2 an. Diese speziellen schweisstechnischen Konstruktionsmassnahmen erlauben eine perfekte gasdichte Verschweissung, obwohl diese zur Vermeidung von Verformungen des sehr dünnwandigen und durch die Abdunstlöcher 1c geschwächten Abdunstkammerteils 1 mit sehr geringer Schweissenergie durchgeführt werden muss. Infolge der Ausrichtung und der geringen Dicke der Schweissrippe 1k wird im Schweisskontaktbereich ein optimales Schwingungspotential wirksam, wodurch sich die Schweissrippe 1k federnd gegen den Absatz 2d drückt und sich bei gleichzeitiger Verformung gasdicht mit diesem verbindet.

Zwischen der Verschweissung 6 und der Einschubdichtungsnute 2b befindet sich noch eine schmale, durch die Anformung der Schweissrippe 1k und einer weiteren Rippe 11 bedingte umlaufende Nute 1m.

Die erzielten Dichtungsverhältnisse sind folgende:

Wird das leicht auf dem Abdunstkammerteil 1 gleitende Abdeckorgan 5 in seine Verschlussposition geschoben, so gelangt dessen verjüngter Randabschnitt bzw. Oeffnungsbereich 5b in die Einschubdichtungsnute 2b, während er gleichzeitig auf den verstärkten Bereich 1i des Abdunstkammerteils 1 auffährt. Da die Wandungen des Reservekammerteiles 2 aufgrund der Verschweissung 6 und ihrer Lage nur geringfügig, d.h. nur im gewollten Masse nachgeben, liegt der Randabschnitt des Abdeckorgans 5 fest in der Einschubdichtungsnute 2b eingeklemmt, in der noch ein kleiner Hohlraum 1n verbleibt. Als Anschlag für das Abdeckorgan 5 dient der Oeffnungsrand 2e des Reservekammerteils 2.

Vom Abdunstraum 1a aus bestehen somit für das sich in diesem befindende abgedunstete Wirkstoffgas zwei hintereinander geschaltete Dichtflächen: eine innere Dichtfläche zwischen dem verstärkten Wandungsbereich 1i und der Innenfläche des Randabschnitts des Abdeckorgans 5 sowie eine äussere Dichtfläche zwischen der Verjüngung 5b und der Verjüngung 2c. Im Vergleich miteinander weist die innere Dichtfläche eine in der Regel deutlich bessere und auch sicherere Dichtwirkung als die äussere Dichtfläche auf. Falls in den Bereich der inneren Dichtfläche Kondensflüssigkeit gelangt, so kann diese infolge Kapillarwirkung nur bis zu dem die genante Wirkung unterbrechenden Hohlraum 1n vordringen. Die Dichtung ist also selbst in Bezug auf eine Benetzung des inneren Dichtungsbereichs mit kondensiertem Wirkstoff praktisch perfekt.

Durch die gasdichte Verschweissung 6 ist von der Reservekammer 2a her ein Verlust von flüssigem oder auch bereits vergastem Wirkstoff an sich ausgeschlossen. Sollte jedoch einmal eine Verschweissung nicht perfekt sein, so ist die erforderliche Dichtigkeit trotzdem durch die die Kapillarwirkung unterbrechende Nute 1m und die mit diesen in Reihe liegende äussere Dichtfläche zwischen den beiden Verjüngungen 2c, 5b in ausreichendem Masse gegeben.

Insbesondere aus den Figuren 2 bis 4 geht noch hervor, dass aussen auf der Breitseitenwandung 1b zwischen den Längsreihen der Abdunstlöcher 1c noch winzige Abstandsgleitrippen 1p vorgesehen sind. Diese verhindern, dass ein z.B. auf dem Wege der Kondensierung zwischen Abdeckorgan 5 und die Seitenwandung 1b gelangendes Wirkstofftröpfchen infolge der Verschiebung des Abdeckorgans 5 zu einer flächigen Benetzung führen kann. Obwohl eine solche Benetzung in erster Linie auf der Seite der Abdunstlöcher auftreten kann, lassen sich entsprechende Rippen natürlich auch aussenseitig der Seitenwandung 1b' anformen. Alternativ können sich solche Rippen auf den Innenflächen des Abdeckorgans 5 befinden. Wichtig ist, dass die Abstandsgleitrippen eine geringere Höhe (etwa 1/10 -2/10 mm) aufweisen als die Dichtfläche im verstärkten Wandungsbereich 1i am Hohlkörper zumindest gegenüber dessen porösen Bereich erhaben ist, damit die Wirkung dieser Verstärkung erhalten bleibt.

Bei der beschriebenen Vorrichtung ist nur die Breitseitenwandung 1b des Abdunstkammerteiles 1 mit Abdunstlöchern 1c versehen. So ausgebildet, dient das Gerät bevorzugt als Mund- und Naseninhalator (z.B. zur Abdunstung eines die Atemwege erfrischenden, bzw. eines heilwirksamen Wirkstoffes wie Eukalyptusöl bei entzündlichen Erkrankungen der Atemwege, eines Wirkstoffes zum Entwöhnen des Rauchens oder dergl.). Zum Inhalieren wird die Vorrichtung mit ihrer die Abdunstlöcher 1c enthaltenden Seite unmittelbar gegen den Mund oder unter die Nase gehalten. Während des Einatmens dringt Aussenluft durch die nicht von Mund oder Nase abgedeckten Abdunstlöcher in den Abdunstraum 1a, streicht parallel zu den Breitseitenwandungen 1b, 1b' an den Wirkstoffleiterbereichen 4a vorbei und gelangt - intensiv mit Wirkstoff angereichert - in den entsprechenden Atemweg.

Soll die Vorrichtung zum Abdunsten von Parfum dienen, so ist die Abdunstwirkung verständlicherweise am grössten, wenn beide Breitseitenwandung 1b, 1b' mit Abdunstlöchern 1c versehen sind, da bei dieser Verwendung die Luft nicht zwangsweise die Abdunstkammer 1a durchströmt. Als Parfum-Abdunster eignet sich die Vorrichtung mittels eines separaten oder angeformten Sockels (die Reservekammer könnten sich im Sockelbereich befinden) oder mittels eines Stützfusses zum Plazieren auf dem Schreibtisch oder dergl.. Als Sockelgerät kann die Vorrichtung natürlich grösser bemessen sein, als wenn sie zum Mitführen in Taschen von Kleidungsstücken bestimmt ist.

## Patentansprüche

1. Vorrichtung zur Abdunstung von Wirkstoffen mit einem plättchenförmig flachen Hohlkörper mit zwei Breitseitenwandungen, von denen wenigstens eine einen porös ausgebildeten Bereich aufweist und mit einem auf diesem gleitend verschiebbar angeordneten, schieberartigen Abdeckorgan, welches aus einer den porösen Bereich des Hohlkörpers abdeckenden Verschlussstellung in wenigstens eine, diesen Bereich zumindest teilweise freigebende Abdunststellung verstellbar ist, dadurch gekennzeichnet, dass der Hohlkörper (1,2) mit einer umlaufenden, zum Abdeckorgan (5) hin offenen, sich nach aussen erweiternden Einschubdichtungsnute (2b) versehen ist, dass das Abdeckorgan (5) zum Zwecke seines Eingriffs mit einem Randabschnitt in diese Einschubdichtungsnute (Verschlussstellung) an diesem Randabschnitt aussenseitig eine zur Einschubdichtungsnute (2b) gegengleiche Verjüngung (5b) aufweist, und dass zumindest die äussere Oberfläche des Hohlkörpers (1,2) im Bereich der Einschubdichtungsnute und zumindest die innere Oberfläche des Abdeckorgans im Bereich seines Randabschnitts als glatte, in der Verschlussstellung dicht aneinander anliegende Dichtflächen ausgebildet sind.

2. Vorrichtung nach Anspruch 1 dadurch gekennzeichnet, dass der Hohlkörper (1,2) im Bereich der Einsschubdichtungsnute (2b) und seiner darin angeordneten Dichtfläche ringsherum geringfügig verstärkt ist, so dass die genannte Dichtfläche zumindest gegenüber dem porösen Bereich des Hohlkörpers (1,2) etwas erhaben ist.

3. Vorrichtung nach Anspruch 1 oder 2, dadurch gekennzeichnet, dass wenigstens die porös ausgebildete Breitseitenwandung (1b) des Hohlkörpers (1,2) und die auf dieser gleitende Wandung des Abdeckorgans (5) durch winzige Abstandsgleitrippen (1p) auf Abstand gehalten sind.

4. Vorrichtung nach Anspruch 2 und 3, dadurch gekennzeichnet, dass die Abstandsgleitrippen (1p) eine geringere Höhe aufweisen als die Dichtfläche am Hohlkörper (1,2) zumindest gegenüber dessen porösen Bereich erhaben ist.

5. Vorrichtung nach einem der Ansprüche 1 bis 4, dadurch gekennzeichet, dass sich an den verjüngten Randbereich des Abdeckorgans (5) ein nach aussen springender Absatz (5c) anschliesst, welcher in der Verschlussstellung des Abdeckorgans (5) am Oeffnungsrand (2e) der Einschubdichtungsnute (2b) anliegt, wobei der genannte Randbereich in die Einschubdichtungsnute elastisch eingreift und dass die Tiefe der Einschubdichtungsnute (2b) derart bemessen ist, dass in dieser Stellung in ihrem hinteren Bereich ein kleiner umlaufender Hohlraum (1n) verbleibt.

6. Vorrichtung nach einem der Ansprüche 1 bis 5, dadurch gekennzeichnet, dass der Hohlkörper (1,2) aus einem ersten (1) und einem zweiten Teil (2) zusammengesetzt ist, wobei der zweite Teil (2) den ersten Teil (1) mit einem Endabschnitt umgreift und wobei die Einschubdichtungsnute (2b) durch eine innenseitige Verjüngung (2c) am genannten Endabschnitt des zweiten Teils (2) des Hohlkörpers (1,2) gebildet wird.

7. Vorrichtung nach Anspruch 6, dadurch gekennzeichnet, dass der erste (1) und der zweite Teil (2) des Hohlkörpers (1,2) in ihrem Überlappungsbereich mittels einer umlaufenden Schweissnaht (6) miteinander verschweisst sind, dass zur Herstellung der Schweissnaht (6) der erste Teil (1) des Hohlkörpers (1,2) umfangsseitig mit einer umlaufenden dünnen Schweissrippe (1k) versehen ist, die in einen etagenförmigen Absatz (2d) im Endabschnitt des zweiten Teils (2) des Hohlkörpers (1,2) eingreift.

8. Vorrichtung nach Anspruch 7, dadurch gekennzeichnet, dass die Schweissnaht (6) um nicht mehr als die Tiefe der Einschubdichtungsnut (2b) von dieser entfernt angeordnet ist.

9. Vorrichtung nach einem der Ansprüche 6 bis 8, dadurch gekennzeichnet, dass der erste Teil (1) des Hohlkörpers (1,2) im Überlappungsbereich mit dem zweiten Teil (2) des Hohlkörpers (1,2) mit wenigstens einer umlaufenden Sperrnute (1m) versehen ist.

10. Vorrichtung nach einem der Ansprüche 7 bis 9, dadurch gekennzeichnet, dass der Innenraum des Hohlkörpers (1,2) durch eine Querwand (1d) in eine vom seinem ersten Teil umschlossene Abdunstkammer (1a) und eine von seinem zweiten Teil umschlossene Reservekammer (2a) unterteilt ist und dass die Querwand (1d) an das in seinen zweiten Teil (2) hineinragende Ende seines ersten Teiles (1), unmittelbar reservekammerseitig jenseits der umlaufenden Schweissnaht (6) angeformt ist.

11. Vorrichtung nach Anspruch 10, dadurch gekennzeichnet, dass die Querwand (1d) mit mindestens einem Durchtrittskanal (1h) versehen ist, dass sich durch diesen Durchtrittskanal (1h) ein mit seinem einen Ende in der Reservekammer (2a) eingebetteter Wirkstoffleiter (4) in die Abdunstkammer (1a) hinein erstreckt, dass der Wirkstoffleiter (4) von im wesentlichen formstabiler, gradliniger Ausbildung und bezüglich seines Durchmessers derart bemessen ist, dass er sich in der Abdunstkammer (1a) weitgehend berührungsfrei von deren Breitseitenwandungen (1b,1b′) erstreckt und dass die Reservekammer (2a) mit einem saugfähigen Material (3) gefüllt ist.

Fig.1

Fig.2 [Schnitt A-A]

Fig.3 [Schnitt B-B]

Fig.4 [Schnitt C-C]

Fig.5 [Schnitt D-D]

## EUROPÄISCHER RECHERCHENBERICHT

**Europäisches Patentamt**

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | FR-A-2 539 031 (BERGER) <br> * Insgesamt * <br> --- | 1 | A 61 L 9/12 |
| A | FR-A-2 271 135 (CIBA-GEIGY) <br> * Seite 4, Zeile 30 - Zeile 40; Figuren 1,2 * <br> --- | 1 | |
| A | WO-A-8 000 003 (WEICK) <br> ----- | | |

**RECHERCHIERTE SACHGEBIETE (Int. Cl.4)**

A 61 L
A 45 D

Der vorliegende Recherchenbericht wurde für alle Patentansprüche erstellt

| Recherchenort | Abschlußdatum der Recherche | Prüfer |
|---|---|---|
| DEN HAAG | 01-03-1989 | SIGWALT C. |